# EUROPEAN PATENT SPECIFICATION

(11) **EP 3 811 080 B1**
(45) Date of publication and mention of the grant of the patent: **11.02.2026**
(21) Application number: 19731312.5
(22) Date of filing: 21.06.2019
(51) Int. Cl.: G01N 33/68

(54) **IN VITRO METHOD FOR THE DIAGNOSIS OF ECTOPIC PREGNANCY**
IN-VITRO-VERFAHREN ZUR DIAGNOSE VON BAUCHHÖHLENSCHWANGERSCHAFT
PROCÉDÉ IN VITRO POUR LE DIAGNOSTIC DE LA GROSSESSE ECTOPIQUE

(30) Priority: 22.06.2018 EP 18382466
(43) Date of publication of application: 28.04.2021
(73) Proprietor: Universidad de Córdoba, 14005 Córdoba (ES); Fundación IVI Edificio Biopolo - La Fe, 46026 Valencia (ES)
(72) Inventor: ROMERO RUIZ, Antonio, 14004 Córdoba (ES); TENA SEMPERE, Manuel, 14004 Córdoba (ES); AVENDAÑO HERRADOR, María Soledad, 14004 Córdoba (ES); PELLICER MARTÍNEZ, Antonio, 46026 Córdoba (ES); DOMÍNGUEZ HERNÁNDEZ, Francisco, 46026 Córdoba (ES)
(74) Representative: Hoffmann Eitle
(86) International application number: PCT/EP2019/066529
(87) International publication number: WO 2019/243605

(56) References cited:
- WO-A1-2016/001613
- WO-A2-2009/047513
- MARÍA GAYTÁN ET AL: "Expression of KiSS-1 in rat oviduct: possible involvement in prevention of ectopic implantation?", CELL AND TISSUE RESEARCH, SPRINGER, BERLIN, DE, vol. 329, no. 3, 15 May 2007 (2007-05-15), pages 571 - 579, XP019542863, ISSN: 1432-0878, DOI: 10.1007/S00441-007-0426-2
- C. N. JAYASENA ET AL: "Reduced Levels of Plasma Kisspeptin During the Antenatal Booking Visit Are Associated With Increased Risk of Miscarriage", JOURNAL OF CLINICAL ENDOCRINOLOGY AND METABOLISM, vol. 99, no. 12, 1 December 2014 (2014-12-01), US, pages E2652 - E2660, XP055515247, ISSN: 0021-972X, DOI: 10.1210/jc.2014-1953
- FRANCISCO DOMINGUEZ ET AL: "Embryonic miRNA Profiles of Normal and Ectopic Pregnancies", PLOS ONE, vol. 9, no. 7, 11 July 2014 (2014-07-11), pages e102185, XP055620637, DOI: 10.1371/journal.pone.0102185
- Z. ZHAO ET AL: "Circulating MicroRNA miR-323-3p as a Biomarker of Ectopic Pregnancy", CLINICAL CHEMISTRY, vol. 58, no. 5, 1 May 2012 (2012-05-01), pages 896 - 905, XP055620641, ISSN: 0009-9147, DOI: 10.1373/clinchem.2011.179283
- MIURA KIYONORI ET AL: "Pregnancy-associated microRNAs in plasma as potential molecular markers of ectopic pregnancy", FERTILITY AND STERILITY, ELSEVIER SCIENCE INC, NEW YORK, NY, USA, vol. 103, no. 5, 13 March 2015 (2015-03-13), pages 1202, XP029155681, ISSN: 0015-0282, DOI: 10.1016/J.FERTNSTERT.2015.01.041

## Description

### FIELD OF THE INVENTION

The present invention refers to the medical field. Particularly, it refers to an *in vitro* method for the diagnosis of ectopic pregnancy (EP).

### STATE OF THE ART

EP is a life-threatening condition, for which novel screening tools enabling early accurate diagnosis would improve clinical outcomes. EP is defined as the implantation and development of a fertilized ovum elsewhere than in the uterine cavity. The fallopian tube is the most common site for ectopic implantation, which otherwise might occur also in the cervix, ovary, abdomen or even a scar of a previous caesarean section. Approximately, 1.5-2% of all reported pregnancies are extrauterine. Despite substantial improvements in its timely management, EP remains the early pregnancy complication with the highest morbidity and mortality rates. EP can result in tubal rupture if not managed promptly, thereby compromising woman's health and future fertility. Although the overall incidence of EP increased by six-fold between the 1970's and 90's, its associated mortality has substantially fallen, largely due to better diagnosis and treatment before rupture. Nonetheless, EP still represents 9-13% of all pregnancy-related deaths in developed countries and is responsible for almost 75% deaths of the first trimester. The situation is much worse in developing countries. For instance, in Africa, women with EP encounter high fatality rates due to late diagnosis, where it accounts for up to 30% of pregnancy-associated deaths.

It is assumed that EP is multifactorial in origin, with several contributing factors, including morphological or functional alterations of fallopian tube permeability, perturbed chemotactic tubal environment, and/or deregulated tubal motility; the major risks factors for EP being a history of tubal surgery and/or extra-uterine pregnancy. However, in more than half of cases of EP, no risk factor can be identified, suggesting the existence of additional, as yet unknown underlying mechanisms. Current diagnosis of EP is based mainly on transvaginal ultrasonography, together with serial measurements of the E-subunit of human chorionic gonadotropin (β-hCG), although laparoscopy is often needed as method of certainty. Indeed, whilst ultrasound and β-hCG tests are extremely powerful for early detection of pregnancy, and identification of some of its eventual complications, their sensitivity and specificity substantially decrease in the case of pregnancies of unknown location, in which false positive or negative diagnosis may occur. In this context, it is especially crucial to avoid erroneous diagnosis of a pregnancy as non-viable, since it may lead to medical or surgical interventions that could eliminate or severely damage a healthy pregnancy.

WO2009/047513 discloses kisspeptin antagonists for modulating kisspeptin activity in order to treat conditions like ectopic pregnancy. Dominguez et al (vol9(7)2014, PLoS ONE) proposes different miRNas as markers for ectopic pregnancy.

Thus, there is an important clinical need to improve the understanding of the pathophysiological basis of EP and to develop better tools, mainly minimally invasive methods, to perform an accurate early screening or diagnosis of this condition.

### DESCRIPTION OF THE INVENTION

### Brief description of the invention

The present invention is aimed at solving the above cited problems by providing an *in vitro* method (hereinafter the method of the invention) for the screening and/or diagnosis of EP, preferably departing from plasma, serum or blood samples. Precisely, the method of the invention is based on the determination of kisspeptins and/or miR-324-3p in plasma, serum or blood samples, alone or in combination with β-hCG, for the non-invasive early identification or detection of EP.

Kisspeptin is a protein that is encoded by the KISS1 gene in humans. In the present invention, it is shown that circulating kisspeptins gradually increased during the first trimester of normal pregnancy, but were massively reduced in EP. This profile correlates with expression levels of KISS1 in human embryonic/placental tissue, which increased in women with normal gestation undergoing voluntary termination of pregnancy (VTOP; taken as control pregnancies) but remained suppressed in EP. In the present invention, bioinformatic predictions and expression analyses identified miR-27b-3p and miR-324-3p as putative repressors of KISS1 in human embryonic/placental tissue at <12-wks gestation, when expression of both miRNAs was low in VTOP controls, but significantly increased in EP. A significant repressive interaction was documented only for miR-324-3p, occurring at the predicted 3'-UTR of KISS1. Interestingly, circulating levels of miR-324-3p, but not of miR-27b-3p, were dramatically suppressed in EP, despite elevated tissue expression of the pre-miRNA, suggesting defective export in EP. A decision-tree model using kisspeptin and miR-324-3p levels was successful in discriminating EP vs. VTOP, with a receiver-operating characteristic (ROC) AUC of 0.95 ± 0.02 (95% CI). Thus, the result obtained in the present invention indicates a massive down-regulation of KISS1/kisspeptins in early stages of EP, likely via a repressive interaction with miR-324-3p. Our data identify circulating kisspeptins and miR-324-3p as novel biomarkers for accurate for screening and/or diagnosing EP at early gestational ages.

Consequently, the data shown in the present invention unambiguously reveal for the first time that circulating kisspeptin levels are markedly decreased during the first trimester of gestation in EP, with a significant drop being already detectable at very early stages (≤6-weeks). This profile grossly correlated with the expression levels of KISS1 in embryonic/placental tissue, in keeping with a potential placental source. In our study, the observed drop in KISS1 expression might have an impact on the whole process of trophoblast invasion, which is inhibited by locally produced kisspeptins, and might facilitate nidation at an ectopic site.

Thus, the present invention unambiguously demonstrates for the first time the capacity of miR-324-3p to suppress KISS1, thus reinforcing the plausibility that the observed increase in miR-324-3p expression in ectopic embryonic/placental tissue is responsible for reduced expression of KISS1/kisspeptin levels in EP. Moreover, the present invention collectively suggests that reduced kisspeptin levels in ectopic pregnancies are not merely due to defective placentation, but rather the consequence of an orchestrated deregulation of a miR-324-3p/KISS1 pathway in ectopic gestational tissue.

To explore the potential diagnostic value of altered expression of miR-324-3p and miR-27b3p in EP, circulating levels of both miRNAs were measured. Surprisingly, while only a rather modest increase in circulating miR-27b-3p was detected in EP, which was significant only at week-8 of pregnancy, the circulating levels of miR-324-3p were massively suppressed in ectopic gestations, with a significant reduction being already detectable at very early (<6-week) stages. The fact that not only the levels of this mature miRNA but also of its pre-miRNA were significantly increased in ectopic tissue suggests that the drop in circulating miR-324-3p concentrations in EP might be due to defective export of this miRNA from its embryonic/placental source, which may lead to accumulation of miR-324-3p; a phenomenon that may further contribute, via repressive interaction with KISS1, to the reduction in circulating kisspeptins in EP. In any event, from a diagnostic standpoint, the concomitant marked suppression of kisspeptin and miR-324-3p levels offers diagnostic possibilities, which were explored in our study. Thus, a biostatistical algorithm based on plasma levels of both factors allowed constructing a decision-tree model that was successful in discriminating EP vs. control pregnancy. Moreover, combination of β-hCG with these novel factors, especially miR-324-3p, permitted to further increase the discriminating power, with ROC data close to one and the added value that incorporation of complementary markers, such as kisspeptins and miR-324-3p, should permit to nullify the risk of false positives or negatives. All these features reinforce the potential of this method for biochemical triage of pregnancies at risk of ectopic gestation.

In summary, the present invention provides herein conclusive evidence for a novel miR-324-3p/KISS1 regulatory pathway, which is altered and may have pathophysiological implications during early ectopic pregnancy. Our data are also endowed with a promising diagnostic dimension, as determination of kisspeptins and/or miR-324-3p, alone or in combination with β-hCG, may allow improvement of current methods for non-invasive identification of EP, with superior sensitivity and specificity.

Consequently, the first embodiment of the present invention refer to an *in vitro* method for the diagnosis of ectopic pregnancy, which comprises determining the concentration level of at least Kisspeptin in plasma, serum or blood samples obtained from the subject, wherein a reduced concentration level of at least Kisspeptin, as compared with a reference control level measured in subjects with normal gestation, is an indication that the subject is suffering from ectopic pregnancy; or to an *in vitro* method for screening subjects at risk of suffering from ectopic pregnancy which comprises determining the concentration level of at least Kisspeptin in plasma, serum or blood samples obtained from the subject, wherein a reduced concentration level of at least Kisspeptin, as compared with a reference control level measured in subjects with normal gestation, is an indication that the subject is suffering from ectopic pregnancy; wherein the methods of the invention further comprises the determination of the concentration level of miR-324 in plasma, serum or blood samples obtained from the subject, wherein a reduced concentration level of the miR-324, as compared with a reference control level measured in subjects with normal gestation, is an indication that the subject is suffering from ectopic pregnancy.

In a preferred embodiment, the methods of the invention further comprises the determination of the concentration level of β-hCG in plasma, serum or blood samples obtained from the subject, wherein a reduced concentration level of β-hCG, as compared with a reference control level measured in subjects with normal gestation, is an indication that the subject is suffering from ectopic pregnancy.

In a preferred embodiment, the methods of the invention comprises determining the concentration level of at least the protein Kisspeptin and miR-324 in plasma, serum or blood samples obtained from the subject, wherein a reduced concentration level of at least Kisspeptin and miR-324, as compared with a reference control level measured in subjects with normal gestation, is an indication that the subject is suffering from ectopic pregnancy.

In a preferred embodiment, the methods of the invention comprises determining the concentration level of at least the protein Kisspeptin, miR-324 and β-hCG in plasma, serum or blood samples obtained from the subject, wherein a reduced concentration level of at least Kisspeptin, miR-324 and β-hCG, as compared with a reference control level measured in subjects with normal gestation, is an indication that the subject is suffering from ectopic pregnancy.

The second embodiment of the present invention refer to the *in vitro* use of at least the concentration level of Kisspeptin in plasma, serum or blood samples obtained from the subject for the diagnosis of ectopic pregnancy, or for screening subjects at risk of suffering ectopic pregnancy wherein said *in vitro* use further comprises the determination of the concentration level of the miR-324 in plasma, serum or blood samples obtained from the subject.

In a preferred embodiment, said *in vitro* use further comprises the determination of the concentration level of the β-hCG in plasma, serum or blood samples obtained from the subject.

In a preferred embodiment, said *in vitro* use further comprises the determination of the concentration level of at least the protein Kisspeptin and miR-324 in plasma, serum or blood samples obtained from the subject.

In a preferred embodiment, said *in vitro* use further comprises the determination of the concentration level of at least Kisspeptin, miR-324 and β-hCG in plasma, serum or blood samples obtained from the subject.

The third embodiment of the present invention refers to the use of a kit comprising reagents for the determination of the level of concentration of at least Kisspeptin for the diagnosis of ectopic pregnancy, or for screening subjects at risk of suffering ectopic pregnancy wherein the kit further comprises reagents for the determination of miR-324 and/or β-hCG for the diagnosis of ectopic pregnancy, or for screening subjects at risk of suffering ectopic pregnancy.

The fourth embodiment of the present invention refer to an *in vitro* method for the diagnosis of ectopic pregnancy, which comprises determining the concentration level of at least miR-324 in plasma, serum or blood samples obtained from the subject, wherein a reduced concentration level of at least miR-324, as compared with a reference control level measured in subjects with normal gestation, is an indication that the subject is suffering from ectopic pregnancy or to an *in vitro* method for screening subjects at risk of suffering from ectopic pregnancy which comprises determining the concentration level of at least miR-324 in plasma, serum or blood samples obtained from the subject, wherein a reduced concentration level of at least miR-324, as compared with a reference control level measured in subjects with normal gestation, is an indication that the subject is suffering from ectopic pregnancy wherein the methods of the invention further comprises the determination of the concentration level of Kisspeptin in plasma, serum or blood samples obtained from the subject, wherein a reduced concentration level of Kisspeptin, as compared with a reference control level measured in subjects with normal gestation, is an indication that the subject is suffering from ectopic pregnancy.

In a preferred embodiment, the methods of the invention further comprises the determination of the concentration level of β-hCG in plasma, serum or blood samples obtained from the subject, wherein a reduced concentration level of β-hCG, as compared with a reference control level measured in subjects with normal gestation, is an indication that the subject is suffering from ectopic pregnancy.

In a preferred embodiment, the methods of the invention comprises determining the concentration level of at least the protein Kisspeptin and miR-324 in plasma, serum or blood samples obtained from the subject, wherein a reduced concentration level of at least Kisspeptin and miR-324, as compared with a reference control level measured in subjects with normal gestation, is an indication that the subject is suffering from ectopic pregnancy.

In a preferred embodiment, the methods of the invention comprises determining the concentration level of at least the protein Kisspeptin, miR-324 and β-hCG in plasma, serum or blood samples obtained from the subject, wherein a reduced concentration level of at least Kisspeptin, miR-324 and β-hCG, as compared with a reference control level measured in subjects with normal gestation, is an indication that the subject is suffering from ectopic pregnancy.

The fifth embodiment of the present invention refer to the *in vitro* use of at least the expression level of miR-324 in plasma, serum or blood samples obtained from the subject for the diagnosis of ectopic pregnancy, or for screening subjects at risk of suffering ectopic pregnancy wherein said *in vitro* use further comprises the determination of the concentration level of Kisspeptin in plasma, serum or blood samples obtained from the subject.

In a preferred embodiment, said *in vitro* use further comprises the determination of the concentration level of the β-hCG in plasma, serum or blood samples obtained from the subject.

In a preferred embodiment, said *in vitro* use further comprises the determination of the concentration level of at least the protein Kisspeptin and miR-324 in plasma, serum or blood samples obtained from the subject.

In a preferred embodiment, said *in vitro* use further comprises the determination of the concentration level of at least Kisspeptin, miR-324 and β-hCG in plasma, serum or blood samples obtained from the subject.

The sixth embodiment of the present invention refers to the use of a kit comprising reagents for the determination of the level of concentration of at least miR-324 for the diagnosis of ectopic pregnancy, or for screening subjects at risk of suffering ectopic pregnancy wherein the kit further comprises reagents for the determination of Kisspeptin and/or β-hCG for the diagnosis of ectopic pregnancy, or for screening subjects at risk of suffering ectopic pregnancy.

The seventh embodiment of the present invention refers to the above cited methods for the diagnosis of ectopic pregnancy, or for screening subjects at risk of suffering ectopic pregnancy, wherein the clinical result obtained by using Kisspeptin and miR-324 is further confirmed by using other techniques, preferably image techniques. In a preferred embodiment, the image technique used in combination with the determination of the concentration levels of Kisspeptin and miR-324 is ultrasonography, preferably transvaginal, endovaginal and/or abdominal ultrasonography.

Such as it is explained above, and it is demonstrated in the examples and figures of the present invention, miR-324 can be used in combination with Kisspeptin and/or β-hCG as a reliable biomarker for the diagnosis of ectopic pregnancy or for screening subjects at risk of suffering from ectopic pregnancy.

Consequently, the eight embodiment of the present invention refers to an *in vitro* method for the diagnosis of ectopic pregnancy, which comprises determining the expression or concentration level of at least miR-324 in plasma, serum or blood samples obtained from the subject, wherein a reduced expression or concentration level of at least miR-324, as compared with a reference control level measured in subjects with normal gestation, is an indication that the subject is suffering from ectopic pregnancy or to an *in vitro* method for screening subjects at risk of suffering from ectopic pregnancy which comprises determining the expression or concentration level of at least miR-324 in plasma, serum or blood samples obtained from the subject, wherein a reduced expression or concentration level of at least miR-324, as compared with a reference control level measured in subjects with normal gestation, is an indication that the subject is suffering from ectopic pregnancy wherein the above cited method further comprises the determination of the concentration level of Kisspeptin in plasma, serum or blood samples obtained from the subject, wherein a reduced concentration level of Kisspeptin, as compared with a reference control level measured in subjects with normal gestation, is an indication that the subject is suffering from ectopic pregnancy.

In a preferred embodiment the above cited method further comprises the determination of the concentration level of β-hCG in plasma, serum or blood samples obtained from the subject, wherein a reduced concentration level of β-hCG, as compared with a reference control level measured in subjects with normal gestation, is an indication that the subject is suffering from ectopic pregnancy.

In a preferred embodiment the above cited method comprises determining the expression or concentration level of at least the protein Kisspeptin and miR-324 in plasma, serum or blood samples obtained from the subject, wherein a reduced concentration level of at least Kisspeptin and miR-324, as compared with a reference control level measured in subjects with normal gestation, is an indication that the subject is suffering from ectopic pregnancy.

In a preferred embodiment the above cited method further comprises determining the expression or concentration level of at least the protein Kisspeptin, miR-324 and β-hCG in plasma, serum or blood samples obtained from the subject, wherein a reduced concentration level of at least Kisspeptin, miR-324 and β-hCG, as compared with a reference control level measured in subjects with normal gestation, is an indication that the subject is suffering from ectopic pregnancy.

The ninth embodiment of the present invention refers to the *in vitro* use of the expression or concentration level of at least miR-324 in plasma, serum or blood samples obtained from the subject for the diagnosis of ectopic pregnancy, or for screening subjects at risk of suffering ectopic pregnancy wherein the above cited use further comprises the determination of the concentration level of Kisspeptin in plasma, serum or blood samples obtained from the subject.

In a preferred embodiment the above cited use further comprises the determination of the concentration level of β-hCG in plasma, serum or blood samples obtained from the subject.

In a preferred embodiment the above cited use further comprises the determination of the expression or concentration level of at least the protein Kisspeptin and miR-324 in plasma, serum or blood samples obtained from the subject.

In a preferred embodiment the above cited use further comprises the determination of the concentration level of at least Kisspeptin, miR-324 and β-hCG in plasma, serum or blood samples obtained from the subject.

The tenth embodiment of the present invention refers to the use of a kit comprising reagents for the determination of the level of expression or concentration of at least miR-324 for the diagnosis of ectopic pregnancy, or for screening subjects at risk of suffering ectopic pregnancy wherein the kit further comprises reagents for the determination of the concentration level of Kisspeptin and/or β-hCG for the diagnosis of ectopic pregnancy, or for screening subjects at risk of suffering ectopic pregnancy.

The eleventh embodiment of the present invention refers to a kit adapted for the diagnosis of ectopic pregnancy, or for screening subjects at risk of suffering ectopic pregnancy, which comprises: a) reagents or means for the determination of the expression or concentration level of miR-324 and b) reagents or means for the determination of the concentration level of Kisspeptin and/or c) reagents or media for the determination of the concentration level of β-hCG.

The last embodiment of the present invention refers to a method for treating patients suffering from ectopic pregnancy once said patients have been diagnosed by using any of the above cited methods.

For the purpose of the present invention the following terms are defined:
- The term "reference control level", when referring to the concentration level of the biomarkers (miRNAs or proteins) described in the present invention, refers to the concentration level observed in women with normal gestation undergoing voluntary termination of pregnancy (VTOP; taken as control pregnancies). The subject is likely to suffer from EP with a given sensitivity and specificity if the concentration level of the biomarker (miRNAs or proteins) is statistically below said "reference control level". A "reference" value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-speciftcity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0.

- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- "Screening" as used herein refers to methods used to evaluate a subject for EP or an increased risk of developing EP. It is not required that the screening procedure be free of false positives or false negatives, as long as the screening procedure is useful and beneficial in determining which of those individuals within a group or population of individuals have EP are at increased risk of EP, and/or are at increased risk of developing EP. A screening procedure can be carried out for both prognostic and diagnostic purposes (i.e., prognostic methods and diagnostic methods).
- "Diagnostic method" as used herein refers to methods carried out on a subject to determine if the subject has EP. Such a subject can be someone having no known risk factors, or someone who may be at risk or has previously been determined to be at risk for a particular neurodegenerative disorder due to the presentation of symptoms or the results of a screening test or other type of diagnostic test.

### Description of the figures

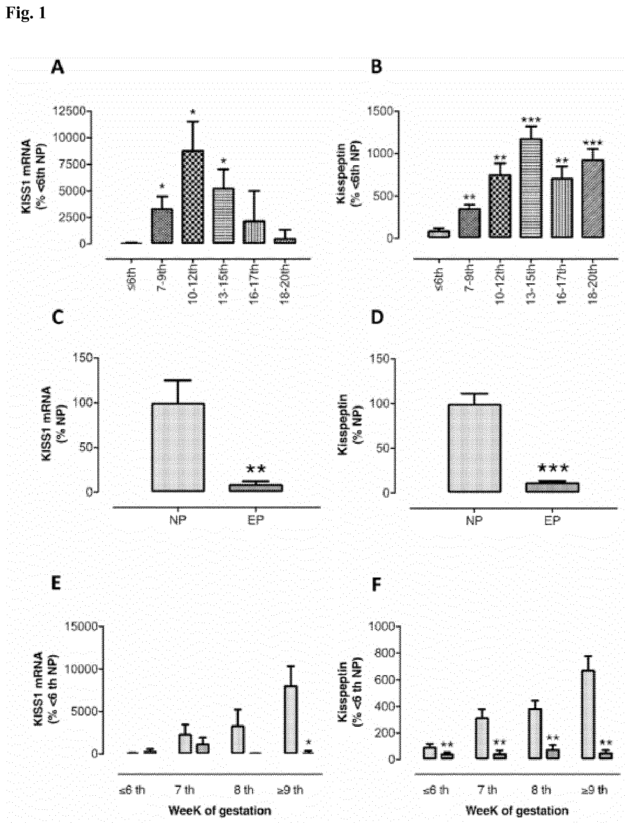

**Figure 1****.** Expression of KISS1 in embryonic/placental tissue and plasma kisspeptin levels in normal (NP) and ectopic (EP) pregnancy. In panels **A)** and **B),** expression of KISS1 and kisspeptin levels in NP (from VTOP) are presented, grouped in six gestational-age ranges: d6th, 7th-9th, 10th12th, 13th-15th, 16th-17th and 18th-20th weeks of pregnancy. Analyses in EP were restricted to samples collected until week 12th of ectopic gestation. Integral mean levels of KISS1 expression and plasma kisspeptin levels in samples from NP and EP up to week-12 of gestation are shown in panels **C)** and **D);** EP values are expressed as normalized values against NP levels. In addition, the detailed temporal course of these changes is shown in panels **E)** and **F).** Samples from week-5 -6 of gestation were grouped as d6-week, whereas those from week -9 to -12 were grouped as t9-week samples. Data are presented as mean r SEM. For presentation, quantitative values were normalized to values from d6-week gestational samples. *, P<0.05; **, P < 0.01; and ***, P < 0.001 vs. corresponding values in NP (ANOVA followed by Newman-Keuls test).

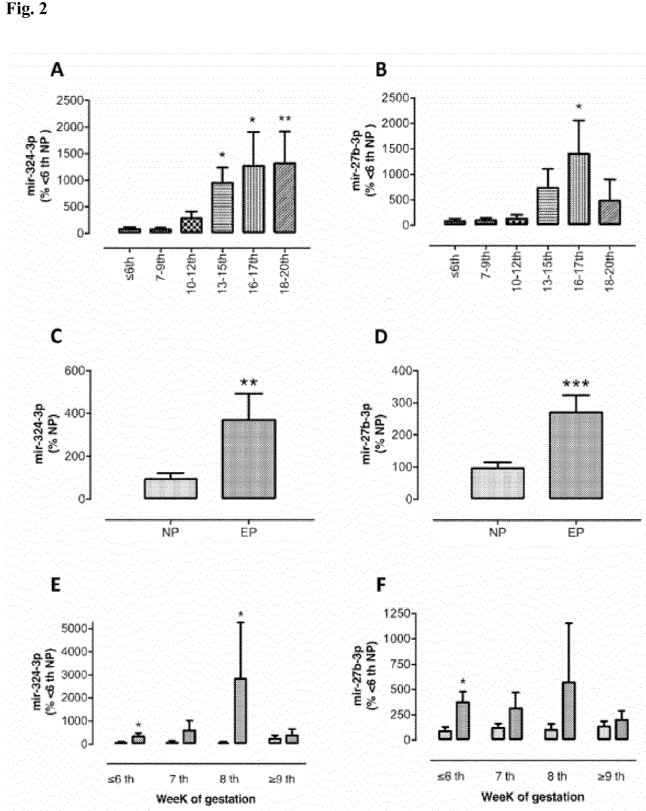

**Figure 2****.** Expression of miR-324-3p and miR-27b-3p in embryonic/placental tissue in normal and ectopic (EP) pregnancy. In panels **A)** and **B),** expression levels of miR-324-3p and miR-27b-3p in embryonic/placental tissue from control pregnancies (NP; from VTOP) are presented, grouped in six gestational-age ranges: d6th, 7th-9th, 10th-12th, 13th-15th, 16th-17th and 18th-20th weeks of pregnancy. Analyses in EP were restricted to samples collected until week 12th of ectopic gestation. Integral mean levels of miR-324-3p and miR-27b-3p in samples from NP and EP up to week-12 of gestation are shown in panels **C)** and **D);** EP values are expressed as normalized values against NP levels. In addition, the detailed temporal course of these changes is shown in panels **E)** and **F).** Samples from week-5 -6 of gestation were grouped as d6-week, whereas those from week -9 to -12 were grouped as t9-week samples. Data are presented as mean r SEM. For presentation, quantitative values were normalized to values from d6-week gestational samples. *, P<0.05; **, P < 0.01; and ***, P< 0.001 vs. corresponding values in NP (ANOVA followed by Newman-Keuls test).

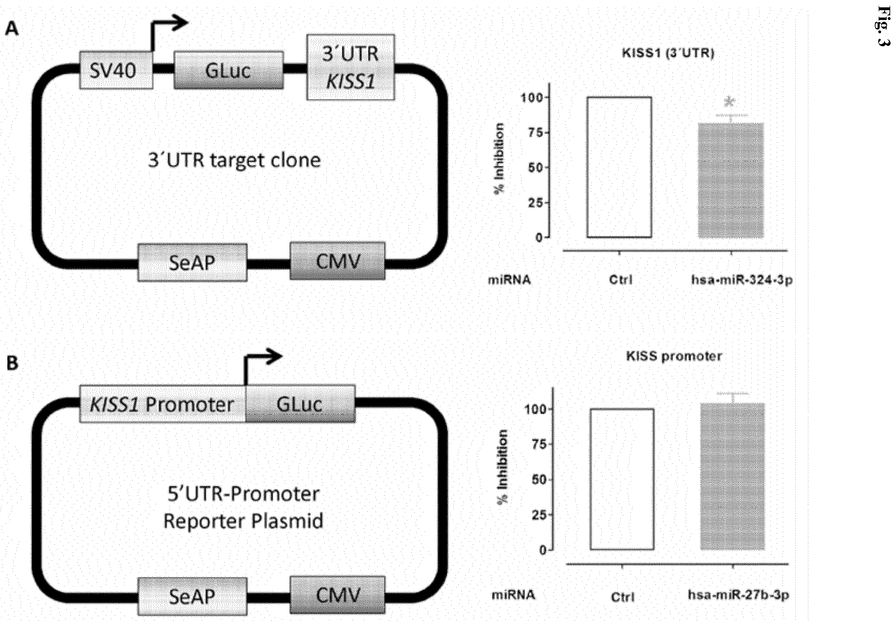

**Figure 3****.** Potential interaction of miR-324-3p and miR-27b-3p with KISS1 *in vitro.* HEK-293T cells were co-transfected with one of the following reporter plasmid: KISS1-3'UTR-Gluc panel **A)** or KISS1-promoter-GLuc panel **B),** together with a plasmid encoding the indicated miRNA (miR324-3p or) miR-27b-3p, or a miRNA scrambled control. Luciferase activity was measured as relative light units (RLU) and is presented as % of inhibition with respect to control values for each miRNA. Data are presented as the mean r SD of n = 3 experiments, done in triplicate. *, P < 0.05 (Student-t test).

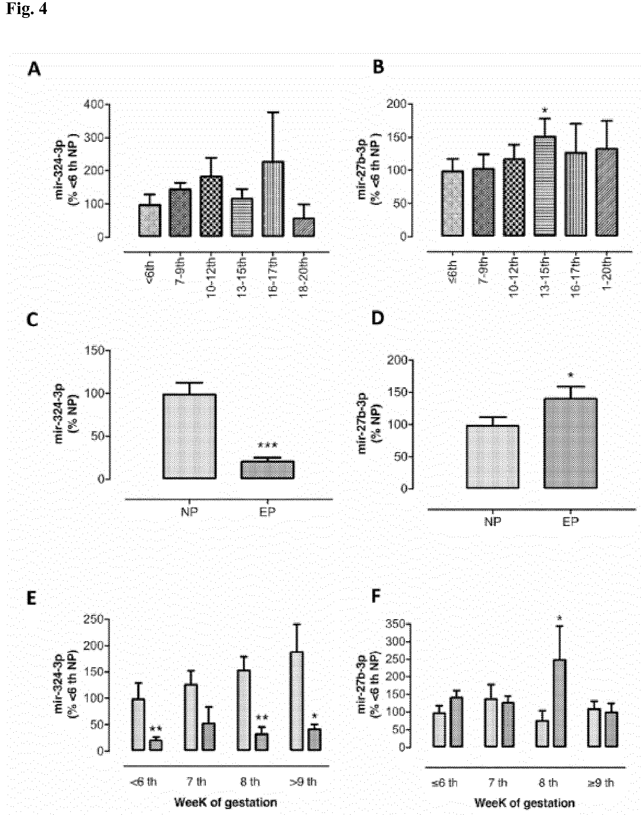

**Figure 4****.** Plasma levels of miR-324-3p and miR-27b-3p in normal and ectopic (EP) pregnancy. In panels **A)** and **B),** plasma levels of miR-324-3p and miR-27b-3p in samples from control pregnancies (NP; from VTOP) are presented, grouped in six gestational-age ranges: d6th, 7th-9th, 10th-12th, 13th-15th, 16th-17th and 18th-20th weeks of pregnancy. Analyses in EP were restricted to samples collected until week 12th of ectopic gestation. Integral mean plasma levels of miR-324-3p and miR-27b-3p in samples from NP and EP up to week-12 of gestation are shown in panels **C)** and **D);** EP values are expressed as normalized values against NP levels. In addition, the detailed temporal course of these changes is shown in panels E and F. Samples from week-5 -6 of gestation were grouped as d6-week, whereas those from week -9 to -12 were grouped as t9week samples. Data are presented as mean r SEM. For presentation, quantitative values were normalized to values from d6-week gestational samples. *, P<0.05; and **, P < 0.01 vs. corresponding values in NP (ANOVA followed by Newman-Keuls test).

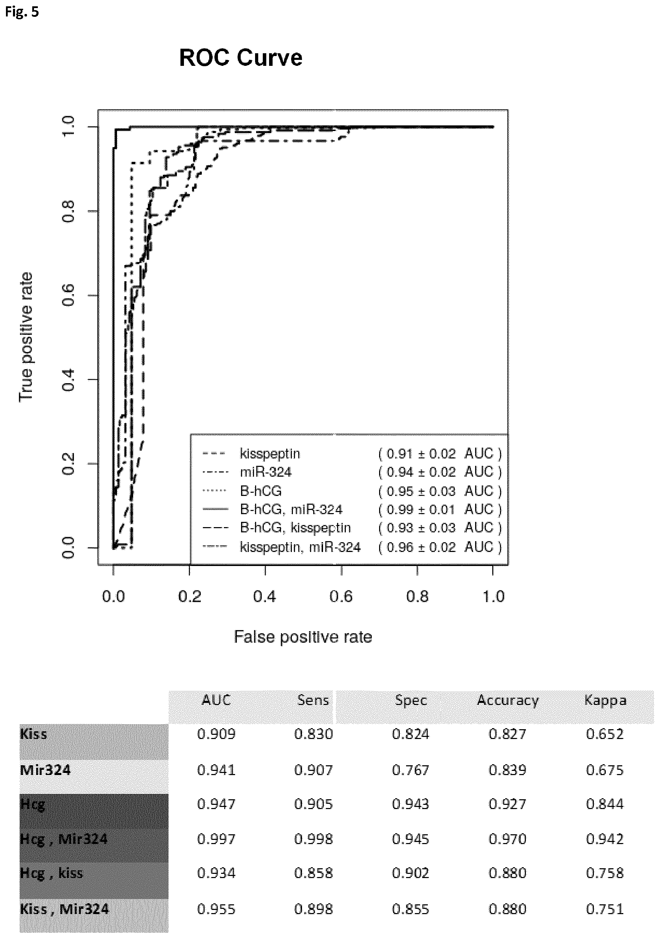

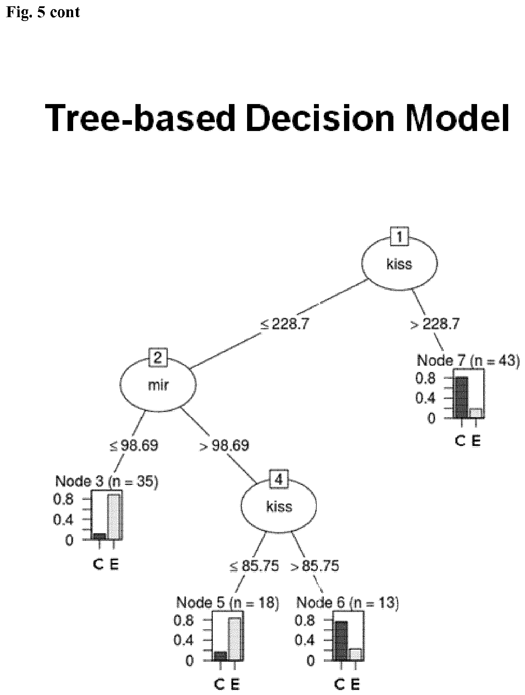

**Figure 5****.** Plasma kisspeptin and miR-324-3p levels as diagnostic markers of EP. In panel **A),** ROC curve analyses of kisspeptins, miR-324-3p, β-hCG, and their combinations in predicting EP. In lower-left panel, performance measures of the corresponding logistic regression models are presented. In addition, in panel **B),** a decision-tree model, generated using miR324-3p and kisspeptin levels, for predicting EP, is shown.

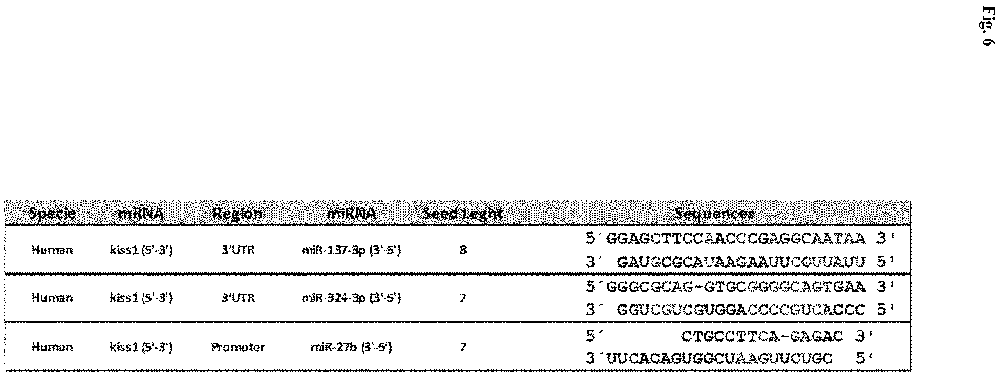

**Figure 6****.** Bioinformatic prediction of putative miRNA regulators of human KISS1. Bioinformatic tools were applied to identify conserved seed regions of miRNAs that may operate as regulators of KISS1. Three major candidates were identified: miR-324-3p and miR-137-3p, with predicted seed sequences at the 3'-UTR of KISS1, and miR-27b-3p, with a predicted seed region at the promoter (5'-UTR) of the gene. Details about location and sequence of these recognition sites are included.

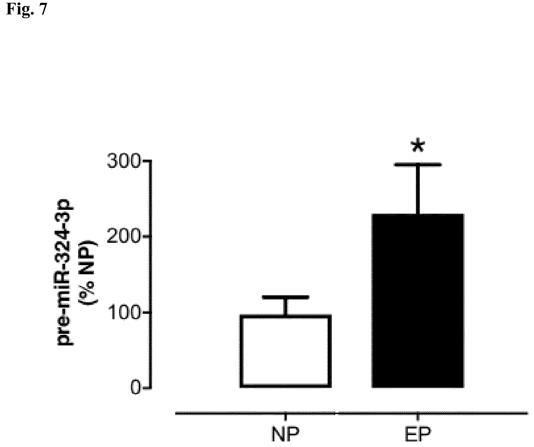

**Figure 7****.** Expression of pre-miR-324-3p in embryonic/placental tissue in normal and ectopic (EP) pregnancy. Integral mean expression levels of pre-miR-324-3p in samples from NP and EP up to week-12 of gestation are shown; EP values are expressed as normalized values against NP levels. *, P < 0.05 (Student-t test).

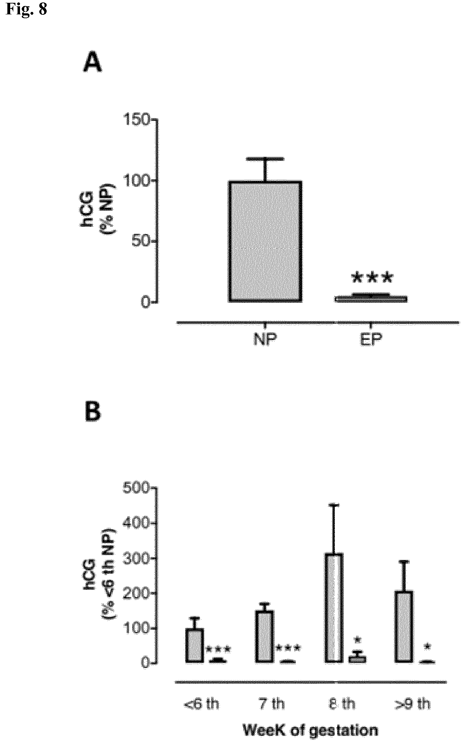

**Figure 8****.** Plasma levels of β-hCG in normal and ectopic (EP) pregnancy. In panel **A),** integral mean plasma levels of β-hCG in samples from NP and EP up to week-12 of gestation are shown; EP values are expressed as normalized values against NP levels. In addition, the detailed temporal course of these changes is shown in panel **B).** Samples from week-5 -6 of gestation were grouped as d6-week, whereas those from week -9 to -12 were grouped as t9-week samples. Data are presented as mean r SEM. For presentation, quantitative values were normalized to values from d6-week gestational samples. *, P<0.05; and ***, P < 0.001 vs. corresponding values in NP (ANOVA followed by Newman-Keuls test).

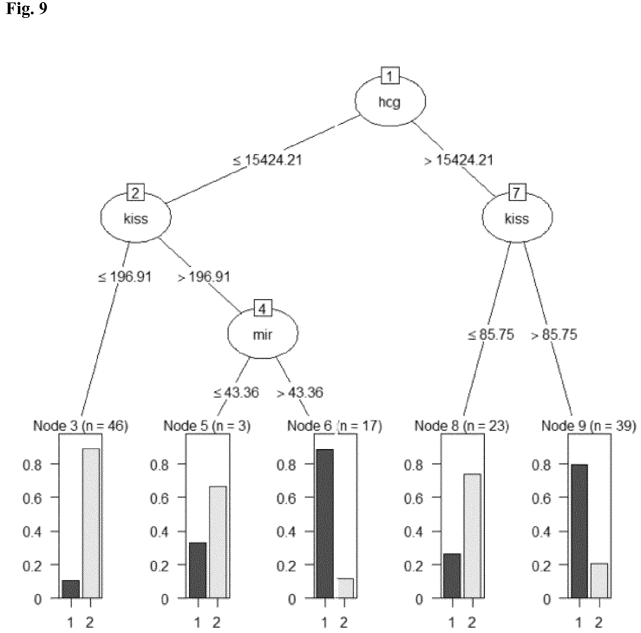

**Figure 9****.** Decision-tree model for triage of EP. A decision-tree model, generated using a sequential combination of β-hCG, kisspeptin and miR-324-3p kisspeptin levels, for predicting EP, is depicted. For further details, see Methods.

### Detailed description of the invention

### Example 1. Ethical Approval.

The present study was approved by the Institutional Review Board/Independent Ethics Committee of the Hospital Universitario La Fe, Valencia, Spain. Early embryonic tissue (mostly trophoblast) was collected after obtaining the corresponding informed consent from each patient, as described elsewhere.

### Example 2. Sample collection.

A total of 108 women with a normal ongoing pregnancy that desired a voluntary termination of pregnancy (VTOP) and 45 patients suffering from tubal ectopic pregnancy were recruited, following previous described criteria. All individuals (EP and VTOP) included in this study signed an informed consent, and samples were properly dated according to the last menstrual period (week of pregnancy). The diagnosis of EP was based on clinical and physical examination, transvaginal ultrasound, and serial quantitative β-hCG levels, and confirmed by laparoscopy in which the tissue was removed, as described previously. The EP patients did not receive methotrexate treatment, and laparoscopy was performed as follows: ectopic pregnancies selected for this study were un-ruptured gestations located in the isthmus or the proximal ampulla. The tube containing the ectopic pregnancy was grasped at both sides (approximately 1 cm away from the gestation site) and bipolar coagulation applied. Similarly, the adjacent mesentery was also coagulated. Then, salpingectomy was performed employing scissors; a longitudinal anti-mesenteric incision into the surface of the tube was made and mild pressure applied with two fingers to extract the gestational sac. Embryonic tissue was carefully separated from obvious blood clots or tubal tissue in the operating room under a stereomicroscope and was immediately placed in TRIzol reagent, frozen and stored at -80°C until use. A piece of each sample was sent to the Pathology Department (Hospital Universitario La Fe, Valencia), in order to provide histological confirmation of ectopic pregnancy and the absence of tubal tissue. The fetal dilation and evacuation method or fetal aspiration technique was performed in VTOP women to obtain the embryonic tissue.

### Example 3. RNA Extraction and Quantitative PCR.

Total RNA was isolated from human embryonic samples from different stages of pregnancy (both control and ectopic gestations), using TRIzol reagent (Invitrogen, CA), following the manufacturer's protocol. The quality and concentration of the isolated RNA were determined by spectrophotometry, following standard procedures. Real-time qPCR was performed on the samples using a Bio-Rad SFX 96 Real-Time System (Bio-Rad Laboratories, Hercules, CA). For quantification of KISS1 mRNA in embryonic samples, 1 µg of total RNA per tissue sample was treated with RQ1 RNAse-free DNAse-I (Promega, Madison,WY) and retro-transcribed (RT) in a 30 µl reaction using iScript^{™} Reverse Transcription Supermix (Bio-Rad Laboratories). For real-time PCR amplification, we used SYBR Green qPCR Master Mix (Promega), with the following primer sequences: hKISS1-forward: 5'-AGC AGC TAG AAT CCC TGG G-3', position 10698-10716 nt (SEQ ID NO: 1); hKISS1-reverse: 5'-AGG CCG AAG GAG TTC CAG T3', position 10947-10929 nt (SEQ ID NO: 2). The primer pair: hL19-forward: 5'-GAA ATC GCC AAT GCC AAC TC-3' (SEQ ID NO: 3) and hL19-reverse: 5'-ACC TTC AGG TAC AGG CTG TG-3' (SEQ ID NO: 4) was used for amplification of a 290-bp fragment of the mRNA of ribosomal L19 protein, which served as internal control for reaction efficiency and sample loading. PCR was initiated by one cycle of 95°C for 2-minutes, followed by 35 cycles of 15-seconds at 95°C, 30-seconds at 62°C, and 10-seconds at 72°C, followed by one final cycle of 72°C for 1-minute. Relative KISS1 mRNA levels were normalized against the expression levels of L19 transcript.

For miRNA analyses, quantification of the expression levels of mir-324-3p and mir-27b-3p was performed according to "miRCURY LNATM Universal RT microRNA PCR individual assay - Instruction manual v6.2", as instructed by Exiqon. All miRNAs were reverse transcribed into cDNA in a single reaction step. The cDNA synthesis control (UniSp6) was added in the reverse transcription reaction giving the opportunity to evaluate the RT reaction. cDNA and SYBR Green qPCR Master Mix (Promega) were transferred to the qPCR plate with specifics primers (Exiqon). The average data obtained from plasma of control pregnancies was used as normalization reference and miR-191-5p was included as housekeeper in qPCR assays for each sample.

### Example 4. Bioinformatic analysis.

Computational miRNA target prediction algorithms were applied to propose putative interactions between the UTR of KISS1 and validated human miRNAs in available databases. Algorithms for predicting miRNAs which can putatively regulate KISS1 were applied to the 3'-UTR and 5' promoter regions, with the following inclusion criteria: (a) miRNAs predicted for interaction at the 3'-UTR had to be identified at least in two of the following databases: http://www.targetscan.org/; http:// www.ebi.ac.uk/enright-srv/microcosm/htdocs/targets/v5/; http://zmf.umm.uni-heidelberg.de/ apps/zmf/mirwalk2/; or http://www.microrna.org/microrna/home/; (b) miRNAs predicted for interaction at the promotor region had to be identified using the database http://mirwalk.umm.uniheidelberg.de; and (c) for all selected targets, conservation of at least 7 nucleotides at the seed region, complementary with KISS1 sequence, was required.

### Example 5. Luciferase reporter assays.

HEK-293T cells (human embryonic kidney cells) were maintained in DMEM supplemented with 10% FBS, 2 mM L-glutamine and 1% (v/v) penicillin/streptomycin at 37 °C in a humidified atmosphere containing 5% CO2. Transient transfections were performed with Rotifect (Carl Roth, Karlsruhe, Germany) according to manufacturer's instructions. The expression vectors for GlucKISS1-3'UTR reporter, hsa-miR-324-3p and hsa-miR-27b-3p, and related reagents, were purchased from GeneCopoeia (Rockville, MD, USA), and luciferase assay performed using Secret-Pair Dual Luminescence Assay Kit according to the manufacturer's instructions (Promega, Madison, WI, USA). For KISS1-3'UTR reporter assays, HEK-293T cells co-transfected with KISS1-3'UTR reporter and hsa-miR-324-3p expression vectors. For KISS1 promoter (5'-UTR) analyses, cells were co-transfected with a KISS1-promoter-Gluc reporter plasmid (KISS1p1339; a kind gift from Dr. Sabine Heger, University of Hanover, Germany) and the hsa-miR-27b-3p expression vector. Cells were collected in PBS after 24h and lysed following the instructions of the luciferase assay kit. Luciferase activity was measured using an Autolumat LB 9510 (EG&G Berthold, USA).

### Example 6. Assays for kisspeptins and β-hCG.

Plasma kisspeptin levels were assayed in control (VTOP) and EP samples using a validated radioimmunoassay; collection of blood samples was done in strict adherence to conditions known to preserve kisspeptins for proper immunometric detection. The kisspeptin antibody, GQ2, which was used at a final dilution of 1:3.500.000, has 100% cross-reactivity with human kisspeptin-54 and shorter fragments (kisspeptin-14 and -10), but <0.01% with any other related human RF-amide peptides. Kisspeptin-54 was labelled with 125I using the Iodogen method. The assay was performed in duplicate using dilutions of neat plasma in 0.7 ml of 0.06 M phosphate buffer, with 0.3% BSA, incubated for 3 days at 4°C. Free and antibody-bound label were then separated by charcoal adsorption. The assay detected changes of 2 pmol/l of plasma kisspeptin IR with a 95% confidence limit. The intra- and inter-assay coefficients of variation were 8.3 and 10.2%, respectively.

For comparative purposes, the levels of β-hCG were also assayed in VTOP and EP samples.

The β-hCG assay was carried out following the instructions of Human β-hCG ELISA Kit from ThermoFisher Scientific (Catalogue No# EHCG). This assay was applied to women with a normal pregnancy that desired a voluntary termination (VTOP) during the first trimester of pregnancy as control (n=35) and to whole cohort of patients suffering from tubal ectopic pregnancy, EP (n=45).

### Example 7. Statistical analyses.

Data of miRNA and kisspeptin levels are expressed as mean ± SEM. Two-tailed Student t tests were used to evaluate differences between case and control groups (single comparisons). ANOVA was used for statistical analyses of multiple data-points; when appropriate post-hoc, Newman-Keuls tests were applied to identify individual differences after multiple comparisons. All statistics were carried out with the use of Prism 7.0c (GraphPad Software, Inc.). P <0.05 was considered to be statistically significant.

Data of patients collected during the first 10 weeks of gestation were used to test the ability of kisspeptins, miR324-3p and β-hCG to predict ectopic pregnancy. Different combinations of these variables were used after logarithmic transformation to build logistic regression models. Due to the limited number of cases data were not divided into training and testing sets. On the contrary, models were built and validated using repeated 10-fold cross-validation. k-fold crossvalidation randomly divides the data into k blocks of roughly equal size. Each block is left out in turn while the other k-1 blocks are used to train the model that is then used to predict the class of the remaining "left-out" subsample. Finally, classification results are summarized into performance measures and averaged to get the overall resampled estimate. Area under the curve, sensitivity, specificity, accuracy (total correct predictions/total samples) and Cohen's (un-weighed) Kappa statistic were computed for each model (kisspeptin, miR324-3p, β-hCG, β-hCG /miR-324-3p, β-hCG /kisspeptin, and kisspeptin/miR-324-3p).

Following the same validation procedure, a decision tree using miR-324-3p and kisspeptin was built. The C50 method (an extension of the C4.5 classification algorithm described in https:// github.com/topepo/caret/) was used for this purpose. All models were built and validated using the R package caret, as defined in http://www.rulequest.com/see5-unix.html.

### Example 8. Embryonic/placental tissue expression of KISS1 and circulating levels of Kisspeptins in EP.

Dynamic profiles of embryonic/placental KISS1 expression and circulating levels of kisspeptins were compared between women with normal gestation undergoing voluntary termination of pregnancy (VTOP; taken as control pregnancies) and women suffering EP. For clinical reasons, we focused our analyses in a gestational window up to 12-weeks. Yet, for reference purposes, in the control group, additional samples from viable pregnancies up to 20 weeks of gestation were also included. Due to the limited number of samples available at early gestational ages, and in order to increase statistical power, samples from 4-, 5- and 6-weeks of gestation were grouped for analysis, as ≤6-week sample. Likewise, in some analyses, data from VTOP samples at the intervals 7-9, 10-12, 13-15 and 16-17 weeks of gestation were pooled together. For the same reasons, samples from control pregnancies at or beyond week-18 of gestation were grouped as 18-20 week samples.

Analysis of KISS1 gene expression in embryonic/placental tissue from women undergoing VTOP revealed negligible expression in ≤6-week samples, with a massive increase thereafter, which reached a maximum >10,000-fold rise at week-12 of gestation. This was followed by a consistent, gradual decline in relative KISS1 levels from gestational week-13 onwards, with relative expression levels that had returned to ≤6-week values in the ≥18-week sample (**Figure 1A**). Circulating levels of kisspeptins followed a grossly similar profile during early gestation, with an ascending curve in the initial weeks of normal pregnancy, and concentrations that increased gradually up to gestational week-15; yet, they remained at a plateau state thereafter (**Figure 1B**).

Similar analyses of KISS1 expression and circulating kisspeptins were conducted in samples from women with EP, collected up to gestational week-12. When calculated as mean values during the whole study period (<12-week), both embryonic/placental KISS1 gene expression and circulating kisspeptin levels were massively suppressed in women with EP vs. VTOP (**Figure 1C-D**). To precisely monitor the timing of such suppression, data analysis was split in ≤6-, 7-, 8- and ≥9week of gestation. In terms of KISS1 mRNA in embryonic/placental tissue, expression levels were equally negligible in the ≤6-week groups from VTOP and EP; yet, from week-7 onward, control pregnancies showed higher KISS1 mRNA levels than EP, although differences were in the limit of statistical significance up to week-9 of pregnancy (Fig. 1E). Circulating levels of kisspeptins followed globally similar profiles, although in this case, plasma concentrations of kisspeptins were significantly lower in EP groups all through the study period, from ≤6-week until week-12 of pregnancy (**Figure 1F**).

### Example 9. Embryonic/placental tissue expression of miR-324-3p and miR-27b-3p in EP.

In an attempt to discover putative regulators responsible for the suppression of KISS1 expression in EP, bioinformatics was applied to identify potential miRNA regulators, by identification of their corresponding seed regions in the 3'-UTR (or eventually 5'-UTR) of the KISS1 gene. By searching different databases and implementing appropriate tools, three major putative candidates were identified: miR-137-3p and miR-324-3p, for which 8 and 7 nucleotides, respectively, from their predicted seed regions were found in the 3'-UTR of KISS1, and miR-27b3p, with 7 nucleotides of its seed region complementary to KISS1 promoter sequence (**Figure 6**). Expression analyses were subsequently applied to evaluate the levels of these miRNAs at early stages of gestation. These analyses documented that while miR-324-3p and miR-27b-3p are readily detectable in human embryonic/placental tissue during the first trimester of gestation; miR-137 is not, therefore suggesting negligible expression of this miRNA, which was excluded from further analyses.

Detailed expression analyses in embryonic/placental tissue from control pregnancies during the first 20-weeks of gestation demonstrated roughly similar expression profiles for both miR-324-3p and miR-27b-3p, with rather low relative expression levels during early stages of gestation, and sharp increase after week-10 (miR-324-3p) or week 13 (miR-27b-3p) of pregnancy (**Figure 2 A-B**). Similar analyses revealed that the expression levels of both miRNAs are significantly elevated in embryonic/placental tissue from EP that, when calculated as mean values during the whole study period (<12-week), represented a 4-fold (miR-324-3p) and 3-fold (miR-27b-3p) increase vs. VTOP levels (**Figure 2 C-D**). Timed analysis of such differences revealed that elevated expression of both miRNAs mainly concentrated at early stages, up to week-8 of gestation, and were already significant at the <6-week group (**Figure 2 E-F**).

### Example 10. Regulation of KISS1 Expression by miR-324-3p and miR-27b-3p.

Based on bioinformatic predictions on the location of seed regions of miR324-3p and miR27b-3p at the 3'- and 5'-UTR of the KISS1 gene, respectively, and the reciprocal changes in expression levels of these miRNAs and their putative target (KISS1/kisspeptins) in EP, we sought to demonstrate whether a direct repressive interaction actually exists, using proper luciferase reporter assays *in vitro.* For testing miR-324-3p/KISS1 interactions, HEK-293T cells were cotransfected with Gluc-KISS1-3'UTR and miR-324-3p reporter plasmids; the former harboring the KISS1 3'-UTR containing the putative miR324-3p seed region downstream the coding sequence of luciferase. As shown in **Figure 3A****,** over-expression of miR324-3p induced a significant 30% drop in luciferase activity, therefore demonstrating a direct interaction and negative post-transcriptional regulation of KISS1 gene by miR-324-3p at its 3'-UTR. In contrast, given the predicted location of the seed region of miR-27b-3p at the 5'-UTR of KISS1, a reporter construct harboring the human KISS1 promoter region upstream the luciferase coding sequence was used. HEK-293T cells were co-transfected with KISS1-promoter-GLuc and miR-27b-3p expression vectors. However, as shown in **Figure 3B****, no** significant changes in luciferase activity were found after miR-27b-3p overexpression in this heterologous cell system.

### Example 11. Circulating levels of miR-324-3p and miR-27b-3p in EP.

Collectively, our results suggested that miR-324-3p and miR-27b-3p are deregulated in EP, and these changes (especially for miR-324-3p) may be mechanistically relevant for the observed suppression of KISS1/kisspeptin in ectopic gestations. To ascertain whether tissue alterations translate into detectable changes in the circulating levels of these miRNA, qPCR analyses were applied to plasma samples from VTOP and EP during the early gestational window. Circulating levels of miR-324-3p and miR-27b-3p were detectable in control (VTOP) pregnant women all through the study period, up to week-20 of gestation, with rather stable values (**Figure 4 A-B**); only a marginal increase in miR-27b-3p levels was detected in 13- to 15-week samples (**Figure 4B**).

Integral plasma levels of miR-324-3p, calculated as mean values during the <12-week period, were significantly suppressed in EP vs. corresponding VTOP levels, with a marked 80% drop in mean concentrations (**Figure 4C**). This substantial drop is in contrast with the observed increase in tissue levels of miR324-3p in EP, which are clearly elevated. To further document this discrepancy, the expression levels of the precursor, pre-miR324-3p, were assayed in <12-week samples from VTOP and EP. In line with the expression profile of mature miRNA, pre-miR324-3p levels in EP were significantly increased (**Figure 7**), therefore confirming the divergence between tissue expression and circulating levels of this miRNA. Opposite to miR-324-3p, mean plasma levels of miR-27b-3p were moderately increased in EP during the <12-week period (**Figure 4D**), which parallels the expression data of the mature miRNA.

Timed analysis of the above changes revealed that circulating levels of miR-324-3p are consistently suppressed in EP all through the early gestational window (grouped at ≤6-, 7-, 8- and ≥9-week samples); yet, changes were in the limit of statistical significance at week-7 (**Figure 4E**). In clear contrast, no significant changes in the plasma levels of miR-27b-3p were detected between control and EP, except for a transient, modest increase in EP at gestational week-8 (**Figure 4F**).

### Example 12. Circulating kisspeptin/miR-324-3p as new early biomarker of EP.

Based on the above data, biostatistics tools were used to study the usefulness of circulating kisspeptins and miR-324-3p as early diagnostic biomarkers of EP, measured alone or in combination. For validation purposes, β-hCG levels, as gold-standard for biochemical diagnosis of EP, were also measured in a representative set of VTOP and EP patients of our cohort. As shown in **Figure 8****,** β-hCG concentrations were markedly suppressed in EP vs. VTOP, when calculated as mean levels over the <12-week gestational period; a profile that resembles that of kisspeptins and miR-324-3p. Time analysis at the ≤6-, 7-, 8- and ≥9-week revealed similar trends, with a consistent suppression of β-hCG concentrations in EP patients, at all time-points studied.

Logistic regressions were applied to evaluate the prognostic power of kisspeptin, miR-324-3p, β-hCG, and their combinations in predicting EP, during the first 12 weeks of gestation. Logistic regression models were built using the R package caret and validated with repeated 10-fold cross-validation. Prediction scores demonstrated the capacity of kisspeptins and miR-324-3p to correctly discriminate between EP and VTOP groups. The overall predictive power of the models is shown in **Figure 5A,** where ROC curves with prediction scores are presented (95% CI). Interestingly, the predictive power of the combination of kisspeptins/miR-324-3p was similar to that of β-hCG, and aggregation of miR-324-3p and β-hCG yielded ROC values close to the unit.

Based on these findings, a decision-tree model using miR-324-3p and kisspeptin levels was constructed using the J48 method implemented in the R package caret and validated with repeated 10-fold cross-validation (0.91 ± 0.01 AUC, 95% CI). In this model, presented in **Figure 5B,** the first node is based on kisspeptin levels (≤228.7 pg/mL, n = 66, or ≥228.7 pg/mL, n = 43), the second node is based on miR-324-3p levels (≤98.69 counts, n = 35, or ≥98.59 counts, n = 31), and the third node is based on kisspeptin levels (≤85.75 pg/mL, n = 18, or ≤85.75 pg/mL, n = 13). In addition, using a similar approach, a decision-tree model incorporating also β-hCG levels was built (**Figure 9**). In this model, the first node is based on β-hCG levels (threshold: 15,424 pg/mL), with a second level of discrimination for values below β-hCG threshold based on kisspeptin levels (threshold: 196.91 pg/mL), and a third level based on miR-324-3p (threshold: 43.46 counts). According to this model, for β-hCG levels over the threshold, discrimination is based on kisspeptin levels, with a nodal point at 85.75 pg/mL.

## Claims

1. *In vitro* method for the diagnosis of ectopic pregnancy or for screening subjects at risk of suffering from ectopic pregnancy which comprises determining the expression or concentration level of miR-324 and Kisspeptin in plasma, serum or blood samples which have been obtained from the subject, wherein a reduced expression or concentration level of miR-324 and Kisspeptin, as compared with a reference control level measured in subjects with normal gestation, is an indication that the subject is suffering from ectopic pregnancy.

2. *In vitro* method, according to claim 1, further comprising the determination of the concentration level of β-hCG in plasma, serum or blood samples which have been obtained from the subject, wherein a reduced concentration level of β-hCG, as compared with a reference control level measured in subjects with normal gestation, is an indication that the subject is suffering from ectopic pregnancy.

3. *In vitro* method, according to any of the previous claims, **characterized in that** it comprises determining the expression or concentration level of the protein Kisspeptin, miR-324 and β-hCG in plasma, serum or blood samples which have been obtained from the subject,
wherein a reduced expression or concentration level of Kisspeptin, miR-324 and β-hCG, as compared with a reference control level measured in subjects with normal gestation, is an indication that the subject is suffering from ectopic pregnancy.

4. *In vitro* use of miR-324 and Kisspeptin expression or concentration levels determined in plasma, serum or blood samples which have been obtained from the subject for the diagnosis of ectopic pregnancy or for screening subjects at risk of suffering ectopic pregnancy.

5. *In vitro* use, according to claim 4, in combination with β-hCG.

6. Kit, adapted for the diagnosis of ectopic pregnancy, or for screening subjects at risk of suffering ectopic pregnancy, which comprises: a) reagents or means for the determination of the expression or concentration level of miR-324 and b) reagents or means for the determination of the concentration level of Kisspeptin.

7. Kit, according to claim 6, further comprising reagents or media for the determination of the concentration level of β-hCG.

## Patentansprüche

1. In-vitro-Verfahren zur Diagnose von Bauchhöhlenschwangerschaft oder zur Screenen von Individuen, welche das Risiko aufweisen, unter Bauchhöhlenschwangerschaft zu leiden, welches das Bestimmen des Expressions- oder Konzentrationsniveaus von miR-324 und Kisspeptin in Plasma-, Serum- oder Blutproben, welche aus dem Individuum erhalten worden sind, umfasst, wobei ein verringertes Expressions- oder Konzentrationsniveau von miR-324 und Kisspeptin, im Vergleich zu einem Referenz-Kontrollniveau gemessen in Individuen mit normaler Gestation, ein Anzeichen dafür ist, dass das Individuum unter Bauchhöhlenschwangerschaft leidet.

2. *In-vitro-Verfahren* nach Anspruch 1, zusätzlich umfassend die Bestimmung des Konzentrationsniveaus von β-hCG in Plasma-, Serum- oder Blutproben, welche aus dem Individuum erhalten worden sind, wobei ein verringertes Konzentrationsniveau von β-hCG, im Vergleich zu einem Referenz-Kontrollniveau gemessen in Individuen mit normaler Gestation, ein Anzeichen dafür ist, dass das Individuum unter Bauchhöhlenschwangerschaft leidet.

3. *In-vitro-Verfahren* nach einem der vorhergehenden Ansprüche, **dadurch gekennzeichnet, dass** es das Bestimmen des Expressions- oder Konzentrationsniveaus vom Protein Kisspeptin, miR-324 und β-hCG in Plasma-, Serum- oder Blutproben, welche aus dem Individuum erhalten worden sind, umfasst, wobei ein verringertes Expressions- oder Konzentrationsniveau von Kisspeptin, miR-324 und β-hCG, im Vergleich zu einem Referenz-Kontrollniveau gemessen in Individuen mit normaler Gestation, ein Anzeichen dafür ist, dass das Individuum unter Bauchhöhlenschwangerschaft leidet.

4. *In-vitro-Verwendung* der miR-324- und Kisspeptin-Expressions- oder Konzentrationsniveaus bestimmt in Plasma-, Serum- oder Blutproben, welche aus dem Individuum erhalten worden sind, zur Diagnose von Bauchhöhlenschwangerschaft oder zur Screenen von Individuen, welches das Risiko aufweisen, unter Bauchhöhlenschwangerschaft zu leiden.

5. In-vitro-Verwendung nach Anspruch 4, in Kombination mit β-hCG.

6. Kit, welches zur Diagnose von Bauchhöhlenschwangerschaft oder zur Screenen von Individuen, welche das Risiko aufweisen, unter Bauchhöhlenschwangerschaft zu leiden, angepasst ist, welches Folgendes umfasst: a) Reagenzien oder Mittel für die Bestimmung des Expressions- oder Konzentrationsniveaus von miR-324 und b) Reagenzien oder Mittel für die Bestimmung des Konzentrationsniveaus von Kisspeptin.

7. Kit nach Anspruch 6, zusätzlich umfassend Reagenzien oder Mittel für die Bestimmung des Konzentrationsniveaus von β-hCG.

## Revendications

1. Procédé *in vitro* pour le diagnostic de la grossesse ectopique ou pour le dépistage de sujets à risque de présenter une grossesse ectopique qui comprend la détermination du niveau d'expression ou de concentration de miR-324 et de kisspeptine dans des échantillons de plasma, de sérum ou de sang ayant été obtenus du sujet, dans lequel un niveau d'expression ou de concentration de miR-324 et kisspeptine réduit, par rapport à un niveau de contrôle de référence mesuré chez des sujets ayant une gestation normale, est une indication que le sujet présente une grossesse ectopique.

2. Procédé *in vitro,* selon la revendication 1, comprenant en outre la détermination du niveau de concentration de β-hCG dans des échantillons de plasma, de sérum ou de sang ayant été obtenus du sujet, dans lequel un niveau de concentration de β-hCG réduit, par rapport à un niveau de contrôle de référence mesuré chez des sujets ayant une gestation normale, est une indication que le sujet présente une grossesse ectopique.

3. Procédé *in vitro,* selon l'une quelconque des revendications précédentes, **caractérisé en ce qu'**il comprend la détermination du niveau d'expression ou de concentration de la protéine kisspeptine, de miR-324 et de β-hCG dans des échantillons de plasma, de sérum ou de sang ayant été obtenus du sujet, dans lequel un niveau d'expression ou de concentration de kisspeptine, miR-324 et β-hCG réduit, par rapport à un niveau de contrôle de référence mesuré chez des sujets ayant une gestation normale, est une indication que le sujet présente une grossesse ectopique.

4. Utilisation *in vitro* de niveaux d'expression ou de concentration de miR-324 et de kisspeptine déterminés dans des échantillons de plasma, de sérum ou de sang ayant été obtenus du sujet pour le diagnostic de la grossesse ectopique ou pour le dépistage de sujets à risque de présenter une grossesse ectopique.

5. Utilisation *in vitro,* selon la revendication 4, en combinaison avec la β-hCG.

6. Kit, adapté pour le diagnostic de la grossesse ectopique, ou pour le dépistage de sujets à risque de présenter une grossesse ectopique, qui comprend : a) des réactifs ou des moyens pour la détermination du niveau d'expression ou de concentration de miR-324 et b) des réactifs ou des moyens pour la détermination du niveau de concentration de kisspeptine.

7. Kit, selon la revendication 6, comprenant en outre des réactifs ou des moyens pour la détermination du niveau de concentration de β-hCG.
